(19) **Europäisches Patentamt**

**European Patent Office**

**Office européen des brevets**

(11) **EP 0 904 056 B1**

(12) ## EUROPEAN PATENT SPECIFICATION

(45) Date of publication and mention
of the grant of the patent:
**01.10.2003  Bulletin 2003/40**

(21) Application number: **97915445.7**

(22) Date of filing: **25.03.1997**

(51) Int Cl.[7]: **A61K 9/00**

(86) International application number:
**PCT/EP97/01560**

(87) International publication number:
**WO 97/036574 (09.10.1997 Gazette 1997/43)**

(54) **PROCESS AND DEVICE FOR INHALATION OF PARTICULATE MEDICAMENTS**

VERFAHREN UND VORRICHTUNG ZUR INHALATION TEILCHENFÖRMIGER MEDIKAMENTE

PROCEDE ET DISPOSITIF DESTINES A L'INHALATION DE MEDICAMENTS PARTICULAIRES

(84) Designated Contracting States:
**AT BE CH DE DK ES FI FR GB GR IE IT LI LU MC
NL PT SE**

(30) Priority: **29.03.1996  GB 9606677**

(43) Date of publication of application:
**31.03.1999  Bulletin 1999/13**

(73) Proprietor: **GLAXO GROUP LIMITED
Greenford, Middlesex UB6 ONN (GB)**

(72) Inventors:
• **VAN OORT, Michiel, Glaxo Wellcome Inc.
Research Triangle Park, NC 27709 (US)**

• **SACCHETTI, Mark J., Glaxo Wellcome Inc.
Research Triangle Park, NC 27709 (US)**

(74) Representative: **Pike, Christopher Gerard et al
Glaxo Wellcome plc,
Glaxo Wellcome House,
Berkeley Avenue
Greenford, Middlesex UB6 0NN (GB)**

(56) References cited:
**EP-A- 0 072 046          WO-A-96/09814**

**Description**

Field of the Invention

The present invention relates, in general, to particulate medicaments and dosing of the medicaments for inhalation by a patient More specifically, the present invention relates to a particulate medicament and the use of the medicament for preparing a medicament formulation, wherein the medicament is in the form of spherical hollow particulates.

Background of the Invention

Asthma and other respiratory diseases are typically treated by the inhalation of an appropriate medicament for deposition into the lungs of a human to ease patient breathing and increase air capacity. Two treatments for respiratory diseases have been widely used. One is inhalation of a medicament from a drug solution or suspension, typically in an aerosol container (i.e., a pressurized container such as a metered dose inhaler) that has a spray valve and uses a gas propellant. The second is inhalation of a powdered drug (generally admixed with an excipient) from a dry powder inhalator.

Manufacture of pressurized aerosol containers filled with medicament and useful as inhalators for respiratory drug delivery is well known, and a representative discussion of such manufacture can be found in Byron, P., Respiratory Drug Delivery, CRC Press, Inc. 185 et seq., (1990) In connection with the manufacture of aerosol inhalators, it is noted that in view of recent evidence of the link between chlorofluorocarbon gas emissions and the deterioration of the earth's protective ozone layer, use of drugs in pressurized aerosol inhalators employing a chlorofluorocarbon (i.e., materials that are totally halogenated with both chlorine and fluorine and thus have no hydrogen on the carbon, for instance, trichloromonofluoromethane, sold by DuPont under the registered trademark FREON 11 and colloquially known as CFC-11, or dichlorodifluoromethane, sold by DuPont under the registered trademark FREON 12 and colloquially known as CFC-12) as the gas propellant has declined. Each of FREON 11 and FREON 12 has an ozone depletion potential (hereinafter, ODP) of 1, and the Environmental Protection Agency of the U.S. Government has imposed regulations to phase out use of such propellants having an ODP = 1.

Instead, environmentally safe propellants having an ODP ≥ 0 and < 0.5 are of increasing interest for use in pressurized aerosol inhalators. Examples of such environmentally safe propellants include, but are not limited to, the following: monochlorodifluoromethane (a hydrochlorofluorocarbon which has an ODP = 0.05 and is sold by DuPont under the registered trademark DYMEL 22); perfluoroethane; 1,1,1,2-tetrafluoroethane (which has an ODP = 0 and is sold by ICI under the trade name HFC-134a); and 1,1,-difluoroethane (which has an ODP = 0 and is sold by various companies under the trade name HFC-152a).

Also, interest in dry powder inhalation systems has increased. Various dry powder inhalator devices for dosing of particulate powdered medicaments to a patient's respiratory tract employ capsules, blisters, velvet fibers, screens, and the like, as a carrier loaded with powdered medicament. For loading the powder in the carrier for the dosing of the powder via an inhalator, typically a selected amount of the powder (such as 50 μg) is admixed with a suspending agent (such as perfluoro-pentane), and the resultant suspension is then dispensed from a metering device to the carrier, after which the suspending agent evaporates and leaves micronized dry powder particles on the carrier.

During use of the inhalator, an air stream (either generated by the patient or by an assist device, as is well known in the art) lifts the powder from the carrier to entrain the powder within the air stream which is then inhaled by the patient. The dose of a powder type of medicament employed with such dry powder inhalator devices is, in most instances, significantly less than 50 mg, typically less than 5 mg, and usually about 50 to about 500 μg. The powdered particles contained in the inhalator are micronized, solid particles, typically having an average particle diameter (colloquially referred to as particle size) of < 10 μm, more particularly < 6 μm, even more particularly < 5 μm, which is an appropriate size so that the particles can be drawn into the lungs.

Representative dry powder inhalator devices having medicament carriers therein and suitable for dispersing of respirable medicaments to patients are disclosed in U.S. Patent Nos. 3,906,950, 4,013,075, 3,807,400, and 3,991,761, each to Cocozza; U.S. Patent No. 4,161,516 to Bell; U.S. Patent No. 4,395,421 to Taylor et al.; European Published Patent Application No. 0 455 463 A1 to Velasquez et al.; European Published Patent Application No. 0 211 595 A2 to Newell et al.; European Published Patent Application No. 0 4670 172 A1 to Cocozza et al.; PCT International Publication No. WO 92/00115, published January 9, 1992, to Gupte et al.; and PCT International Publication No. WO 94/20164, published September 15, 1994, to Mulhauser et al. Also, the commercially available TURBUHALER® inhalator is disclosed in U.S. Patent Nos. 4,667,668 and 4,805,811, each to Wetterlin, and U.S. Patent No. 4,668,218 to Virtanen.

Additionally, it is noted that U.S. Patent No. 5,503,869, issued April 2, 1996, and US Patent Application Serial No. 08/328,578, filed on October 21, 1994, both to Van Oort, describe a medicament carrier which is adapted for use in a dry powder inhalator device and includes at least one carrier screen having carrier surfaces that define a plurality of interstices in the screen. At least one dose of a powdered medicament is loaded onto the carrier screen surfaces whereby the interstices of the screen are at least partially open and free of the powdered medicament. Spray drying for preparation of particles, including for selected medicinal uses, is well known. Additionally, the concept is well known that, under certain conditions during spray drying from solution, the resultant particles are not solid, but rather are hollow structures. Selected uses of such hollow structures involve certain medical applications.

For instance, U.S. Patent 4,590,206 to Forrester et al. shows spray dried respirable medicament particulates, such as sodium cromoglycate, in the shape of doughnut rings, where the hollowness is the hole in the middle of the ring and the ring is solid. Since spray dried hollow spheres have a low particle density, they are considered by Forrester et al. to be too fragile and are consequently to be avoided.

Also, U.S. Patent No. 4,127,622 to Watanabe et al. shows hollow particulates for gastric medicines suspendable in gastric juice and which may remain in the stomach for a long time. They are prepared by dissolving S-PI (substance for pepsin inhibition as the active ingredient) and ethylcellulose (as the excipient) in a lower chlorinated hydrocarbon (as the solvent), so that the concentration of ethylcellulose is 0.5 to 4% by weight on the basis of the total weight of the solution, and then spray drying the solution at a temperature higher than 50°C.

Also, of interest in connection with hollow structures for useful as medicaments is the disclosure of PCT International Publication No. WO 91/12823, published September 5, 1991, to Illum et al. This publication describes hollow (i.e., gas-filled or vapor-filled) microcapsules (for instance, albumin) prepared by forming a shell around a solid or liquid core (for instance the volatile oil, perfluorohexane), and then removing the core. The shell may be made by variations on spray drying, such as simple or complex coacervation, oil/water/oil double emulsion, or MSIEP (minimization of solubility at isoelectric point) methods, followed by chemical or heat hardening to render the shell water insoluble. The double emulsion method results in each microcapsule having a honeycomb appearance with multiple gas-filled chambers. The microcapsules are injected into the blood of a human for use in echocardiography.

Nevertheless, such spray drying techniques to achieve spherical hollow particulate structures for respirable medicaments that are to be inhaled by the patient have not previously been employed.

Summary and Objects of the Invention

Accordingly, the present invention provides a process for dispersing spherical hollow medicament particulates from an inhalator device. The inhalator device may be a dry powder inhalator having contained therein a medicament carrier loaded with at least one dose of dry powdered medicament particles comprising spherical hollow particulates of respirable particle size suitable for deposition in a human being's lungs. Alternatively, the inhalator device may be a pressurized aerosol inhalator, such as a metered dose inhalator, having contained therein a propellant and at least one dose of medicament particles comprising spherical hollow particulates of respirable particle size suitable for deposition in a human being's lungs. For both dispersion from the dry powdered inhalator and from the pressurized aerosol inhalator, the spherical hollow medicament particulates should have a mass median aerodynamic diameter suitable for deposition in a human being's lungs.

Additionally, the present invention provides an inhalator device suitable for dispersing medicament therefrom and containing medicament therein, where the medicament comprises spherical hollow particulates that are of respirable particle size. The inhalator device may be a dry powder inhalator. Alternatively, the inhalator device may be a pressurized aerosol inhalator, such as a metered dose inhalator. For both the dry powdered inhalator and the pressurized aerosol inhalator, the spherical hollow medicament particulates should have a mass median aerodynamic diameter suitable for deposition in a human being's lungs.

It is therefore an object of the present invention to provide a medicament for use in an inhalator which provides for administration of a dosage of medicament particles wherein the particles that leave the inhalator and are inhaled into the patient's lungs are formed as spherical hollow particulates having a desirable aerodynamic particle size and thus are of respirable particles size (i.e., they should have a mass median aerodynamic diameter suitable for deposition in a human being's lungs) for maximum beneficial efficiency, providing maximum efficacy to the patient.

It is an advantage of the present invention that the spherical hollow particulate form can improve the deaggregation properties of the medicament for entrainment in the stream, as the medicament is moving from the inhalator device into the patient's lungs, since the spherical hollow particulates can be made with a large geometric diameter and thus will have less surface-to-surface contact with each other as compared to conventional micronized solid particulates that have a relatively smaller geometric diameter.

Some of the objects and advantages of the invention being stated, other objects will become evident as the description proceeds, when taken in connection with the accompanying Figures and Laboratory Examples described below.

Brief Description of the Figures

Figure 1 is a photomicrograph of spray dried dimpled solid particulates of the asthma medicament, albuterol sulfate;

Figure 2 is another photomicrograph of spray dried dimpled solid particulates of albuterol sulfate;

Figure 3 is a photomicrograph of spray dried spherical hollow particulates of the asthma medicament, amiloride HCl;

Figure 4 is another photomicrograph of spray dried spherical hollow particulates of amiloride HCl;

Figure 5 is another photomicrograph of spray dried spherical hollow particulates of amiloride HCl;

Figure 6 is a photomicrograph of spray dried spherical hollow particulates of the excipient, lactose; and

Figure 7 is another photomicrograph of spray dried spherical hollow particulates of lactose.

Detailed Description of the Invention

It is well known that, during inhalation therapy or systemic absorption via the respiratory tract, the human lung separates particles based on the aerodynamic diameter, which is a function of the actual average particle diameter (i. e., the geometric diameter), as well as the shape and the density of the particle. More specifically, a lower particle density will produce a smaller aerodynamic diameter for particles of equivalent geometric since size, as illustrated by equation 1 as follows:

$$D_{ae} = D_{geo} \, p^{1/2} \qquad \qquad \text{(equation 1)}$$

where $D_{ae}$ and $D_{geo}$ are the aerodynamic and geometric diameters, respectively, and p is the particle density.

Because of the spherical particulates being hollow, they have an actual density lower than that of the solid particulates currently employed for respirable medicaments. Thus, applicants have unexpectedly discovered that the spherical hollow particulates should be perceived by the lung as being of a smaller aerodynamic size than the aerodynamic size of the solid particulates of substantially the same actual average particle diameter, and thus the spherical hollow particulates should be deposited deeper in the lungs.

Moreover, the spherical hollow particulates can be made with an actual average particle diameter (i.e., the geometric diameter) greater than that of the solid particulates currently employed for respirable medicaments. In that event, the spherical hollow particulate form would likely improve the deaggregation properties of the medicament for entrainment in the inhalation stream, as the medicament is moving from an inhalator device into the patient's lungs, due to the large spherical hollow particulates having less surface-to-surface contact with each other as compared to the relatively smaller solid particulates. As a result, an increase in the respirable fraction of a medicament formulation should be achieved with large spherical hollow particulates as compared to small solid particles, where the mass median aerodynamic diameter of the two is approximately the same.

As noted above, methods for spray drying of particles are well known, and it is also well known that controlling selected conditions for spray drying, such as the temperature, the type of solvent, the concentration of the active ingredient and/or the optional excipient, can result in the spray dried particles being hollow structures instead of solid. Any of the various well known spray drying methods may be employed for spray drying the medicament particles in accordance with the present invention to form spherical hollow structures useful for inhalation therapy or systemic absorption via the respiratory tract, including those spray drying methods disclosed in the above-mentioned U.S. Patent 4,590,206 to Forrester et al., U.S. Patent No. 4,127,622 to Watanabe et al. and PCT International Publication No. WO 91/12823 to Illum et al., the disclosures of which are incorporated herein by reference.

Various solvents may be employed during spray drying, including, but not limited to, hydrocarbons, halogenated hydrocarbons (i.e., fluorinated hydrocarbons or chlorinated hydrocarbons), alcohols, ketones, and the like. Examples of suitable solvents include, but are not limited to, water, hexane, perfluoromethylcyclohexane, perfluorohexane, perfluoropentane, dichloromethane, ethanol, acetone, and combinations thereof.

Medicament particles which may be spray dried in accordance with the present invention to form spherical hollow particulates are suitable for use as respirable medicaments for inhalation therapy or systemic absorption via the respiratory tract to treat respiratory disorders such as asthma, bronchitis, chronic obstructive pulmonary diseases and chest infection. Additional medicaments may be selected from any other suitable drug useful in inhalation therapy and which may be presented as a suspension or in a dry powder inhalator. Appropriate medicaments may thus be selected from, for example, analgesics, e.g., codeine, dihydromorphine, ergotamine, fentanyl or morphine; anginal preparations, e.g. diltiazem; antiallergics, e.g. cromoglycate, ketotifen or neodocromil; antiinfectives e.g. cephalosporins, penicillins, stretomycin, sulphonamides, tetracyclines and pentamidine; antihistamines, e.g. methapyrilene anti-inflammatories, e.g. fluticasone, flunisolide, budesonide, tipredane or triamcinolone acetonide; antitussives, e.g. noscapine; bronchodilators, e.g. salmeterol, salmbutamol, ephedrine, adrenaline, fenoterol, formoterol, isoprenaline, metaproterenol, phenylephrine, phenylpropanolamine, pirbuterol, reproterol, rimiterol, terbutaline, isoetharine, tulobuterol orciprenaline, pirbuterol, reproterol, rimiterol, terbutaline, isoetharine, tulobuterol orciprenaline, or (-)-4-amino-3,5-dichloro-$\alpha$-[[[6-[2-(2-pyridinyl)ethoxy]-hexyl]amino]methyl]benzenemethanol; diuretics, e.g. amiloride; anticholinergics, e.g. ipratropium, atropine, oxitropium; hormones, e.g., cortisone, hydrocortisone or prednisolone; xanthines e. g. aminophylline, choline theophyllinate, lysine theophyllinate or theophylline and therapeutic proteins and peptides, e.g. insulin or glucagon. It will be clear to a person skilled in the art that, where appropriate, the medicaments may be used in the form of salts (e.g. as alkali metal or amine salts or as acids addition salts) or as esters (e.g. lower alkyl esters) or as solvates (e.g. hydrates) to optimise the activity and/or stability of the medicament. Preferred medicaments are salbutamol, salmeterol, fluticasone propionate, beclomethasone dipropionate, terbutaline, cromoglycate, budesonide, and triamcinolone acetonide and/or salts thereof.

Moreover, the medicaments optionally may be together with excipients acceptable for inhalation into the human body, which may be organic excipients, such as polysaccharides (i.e., starch, cellulose, and the like), lactose, glucose, mannitol, amino acids, and maltodextrins, or may be inorganic excipients, such as calcium carbonate and sodium chloride. The excipient may be included with the medicament via well known methods, such as by admixing, co-precipitating, and the like.

When entrained in an inhalation stream for inhalation by the patient, the spherical hollow particulates typically should acquire a mass median aerodynamic diameter, particularly from about 0.5 µm to about 7.0 µm, more particularly from about 1 µm to about 4.5 µm, as perceived by the patient's lungs as the spherical hollow particulates pass into the lungs. Also, the spherical hollow particulates typically should have > 50% of the mass of hollow particulates, more particularly > 70% of the mass of hollow particulates, particularly having a mass median aerodynamic diameter < 6 µm, more particularly < 5 µm, as perceived by the patient's lungs as the spherical hollow particulates pass into lungs. As noted in the above discussion of prior art inhalators, it is particularly useful that particles of respirable particle size range have more than 50% thereof with a mass median aerodynamic diameter < 6 µm, more particularly < 5 µm, for appropriate deposition into the lungs, which should be achieved with the present invention.

The ability to control the density of the substantially spherical hollow particulates offers an additional advantage over current inhalator systems which use a suspension of medicament particulates in a propellant. In existing systems containing drug and propellant suspensions, the suspension may separate or stratify because of the differences in the densities of the medicament and propellant.

Separation may be either classified as "creaming" wherein the medicament rises to the top of the more dense propellant, or "sedimentation" wherein the medicament settles to the bottom of the less dense propellant. Regardless of the classification, separation of the medicament and component may cause a lack of dosage uniformity per activation, i.e., each dose may not provide an equal amount of drug over the life a multi-dose inhalator. The uniformity of dosages delivered by multi-dose inhalators is of critical importance to the efficacy of the device and must be within narrow parameters to meet regulatory criteria.

The problem of separation of the suspension is generally addressed by vigorously shaking the inhalator immediately before it is used. However, patient compliance with this simple task is difficult to control and even slight delays between shaking and use effect dosage uniformity.

The present invention, however, overcomes the problem separation and, in so doing, conceivably eliminates the need to shake the inhalator before use. By allowing the drug to be density matched to the selected propellant, the tendency of the medicament and propellant to stratify is removed. The drug and propellant are uniformly distributed in suspension and it can be assumed that each dose would then also be similarly uniform.

Medicament density may be pre-selected and controlled by adjusting the spray drying conditions under which the particulates are created, as previously mentioned. In particular, though, density may be controlled by adjusting the thickness of the walls of the spheres as compared to sphere diameter, and by adjusting the ratio of drug to excipient when creating composite medicament particulates. In some embodiments, however, it may be prefererred to use pure medicaments without excipients. In short, the ability to pre-select and control the density of the medicament particulates offers a significant advantage over existing medicament/propellant suspension systems.

With respect to dry powder inhalators, the spherical hollow particulates of the present invention are suitable for use in any carrier in any dry powder inhalator, including, but not limited to, any of the dry powder inhalators disclosed in the above-mentioned patents and published patent applications.

The spherical hollow particulates of the present invention are also suitable for use in any metered dose inhalator, including the pressurized aerosolized type (where the particulates are together with a propellant and an optional suspending agent). With respect to pressurized aerosol metered dose inhalators, as such pressurized aerosol containers are well known in the art, the spherical hollow particulates may be placed in a pressurized container with a suitable propellant, and optionally with a suitable suspending agent (also known as a dispersing agent or a surfactant) by any of the well known methods therefor, such as that shown in the above-noted Respiratory Drug Delivery, p. 185 et seq. In general, adding a medicament to a pressurized aerosol container is accomplished as follows.

Medicament is added to a high shear blender (i.e., mixer) which contains propellant and may also contain a suspending agent It may also be preferred to add a polar substance to increase solubility of surfactant in a propellant, e.g. ethanol.

Propellants may be of the chlorofluorocarbon variety (i.e., trichloromonofluoromethane, sold by DuPont under the registered trademark FREON 11 and colloquially known as CFC-11, or dichlorodifluoromethane, sold by DuPont under the registered trademark FREON 12 and colloquially known as CFC-12), which, as mentioned above, are being phased out by the Environmental Protection Agency of the U.S. Government as each of FREON 11 and FREON 12 has an ODP = 0. Alternatively, propellants may be of the more recently developed environmentally safe varieties. Suitable environmentally safe propellants include, but are not limited to, any of the above-mentioned perfluoroethane, monochlorodifluoromethane, 1,1,1,2-tetrafluoroethane, 1,1,-difluoroethane, 1,1,1,2,3,3,3-heptafluoro-n-propane, and combinations thereof.

Suitable optional suspending agents include, but are not limited to, oleic acid, SPAN ® 85 (registered trademark for the partial esters of the common fatty acids (lauric, palmitic, stearic, and oleic) and hexitol anhydrides (hexitans and hexides), that are derived from sorbitol and that tend to be oil-soluble and dispersible or insoluble in water, lecithin, and combinations thereof.

If the propellant has a low boiling point so that it would volatilize during procedures at or near room temperature, then the mixer needs to be maintained well below room temperature to prevent evaporation or alternatively a sealed mixer (one in a closed system with the container) may be employed. Once a homogenous suspension is obtained, the suspension is filled into aerosol containers. During the filling, the mixer can be used to maintain adequate suspension throughout the entire filling circuit by continuously circulating the suspension through the concentrated filling unit.

At this point, there exist two main options. With the first option, especially for products not using the environmentally unsafe propellant, CFC-11, the entire formulation is prepared in a low temperature pressure vessel and then filled through the valve into evacuated, previously crimped containers. Care must be taken with propellants such as HFC-134a, that have a high vapor pressure, as filling through the valve of the container is difficult with such propellants. The second option involves the manufacture of a lower volatility concentrate. With this alternative technique, filling is in a controlled environment into containers, after which the valves are crimped in place. Subsequently, the high pressure propellant is added through the valve.

The tensile strength of the spherical hollow particulates will vary depending on the particular medicament (and optional excipient) being spray dried. In the event that the spherical hollow particulates have a weak enough tensile strength so that a large storage container of them, such as a kilogram quantity, would result in upper hollow particulates crushing lower hollow particulates in the container prior to deposition of the hollow particulates in an inhalator device, then formation of hollow particulates should be accomplished in-line so that the formed hollow particulates can be deposited directly after formation into an inhalator device.

Laboratory Examples

Example I

Production of hollow particulates by spray drying.

Medicament powder of each of the two medicaments, albuterol sulfate and amiloride HCI (abbreviated herein as Alb S and Amil HCI, respectively), is employed in this example.

Also, the excipient, lactose, is employed in this example. Aliquots of each of the medicaments, and also of lactose, are spray dried as follows.

15 g of Alb S (lot no. W 1946 FB) are dissolved in 300 ml of water to create a 5% solution. Similarly, 3.479 g of Amil HCI (lot no. 9007H 902) are diluted in water to 1000 ml to create a solution. Likewise, 15 g of lactose (lot no. 1NC25, 605 from Sheffield Products of Norwich, New York) are diluted in water to 150 ml to create a solution.

Each solution is respectively spray dried using a VIRTIS™ (a spray dryer commercially available from The Virtis Company of Gardiner, New York) with each of the air from the nozzle and from the blower set at its respective maximum value under the following conditions of temperature and rate, as summarized in Table A below:

TABLE A

| Spray Dried Particles | Inlet Temp (°C) | Outlet Temp (°C) | Flow Rate Setting (ml/minute) |
|---|---|---|---|
| Alb S (medicament) | 150 | 101 | 7 |
| Amil HCI (medicament) | 150 | 92 | 12 |
| lactose (excipient) | 180 | 127 | 5 |

Spray drying produced the following average particle diameters (i.e., the geometric diameters) as summarized in Table B below:

TABLE B

| Spray Dried Particles | Geometric Diameter | Hollow or Solid |
|---|---|---|
| Alb S | 1 to 5 μm | dimpled solids |
| Amil HCI | 1 to 5 μm | hollow spheres |
| Lactose | 2 μm | hollow spheres |

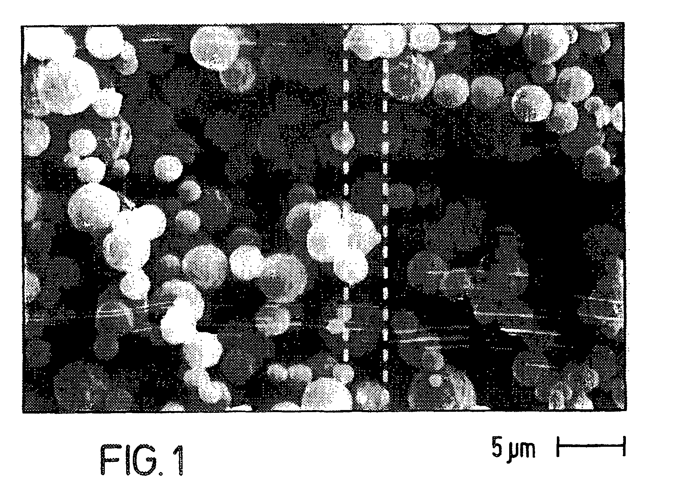

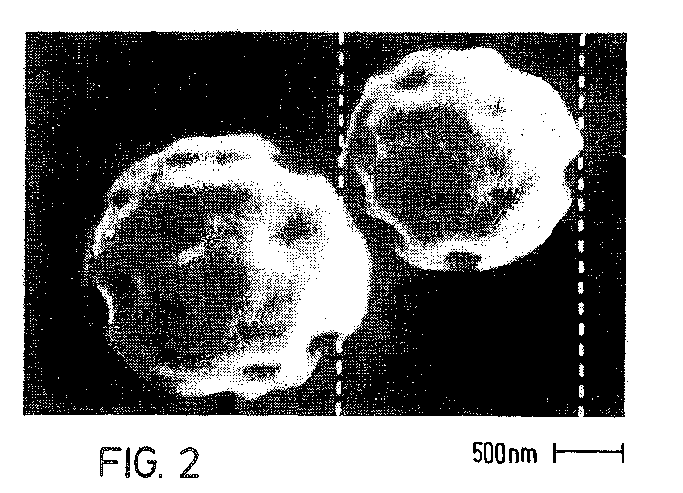

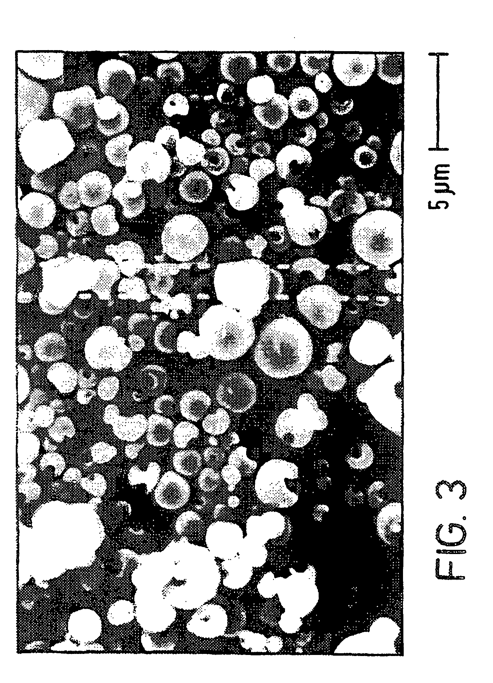

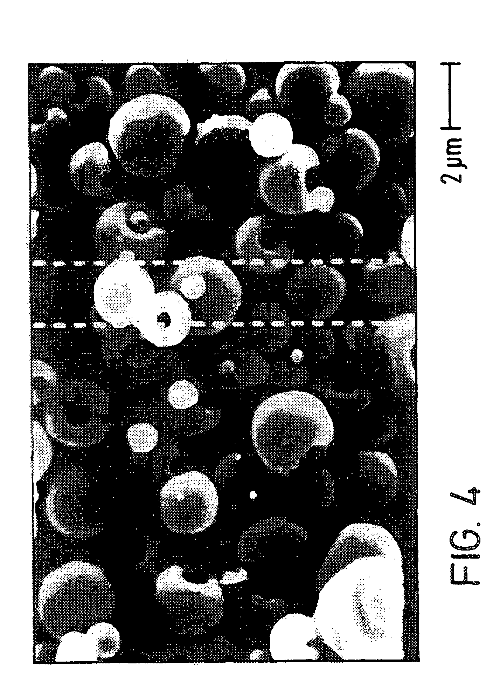

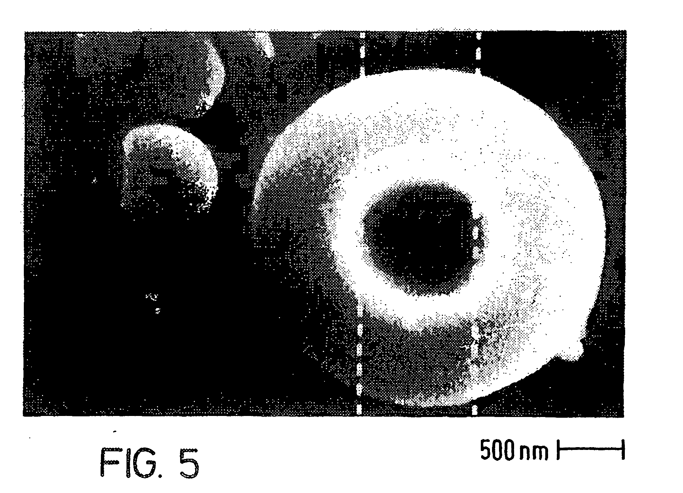

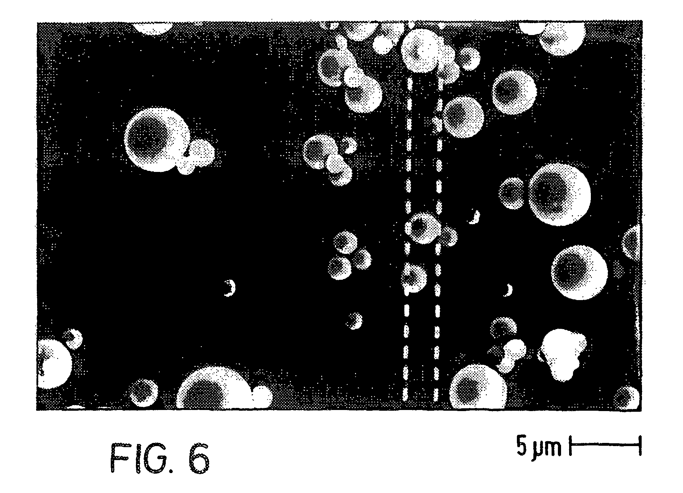

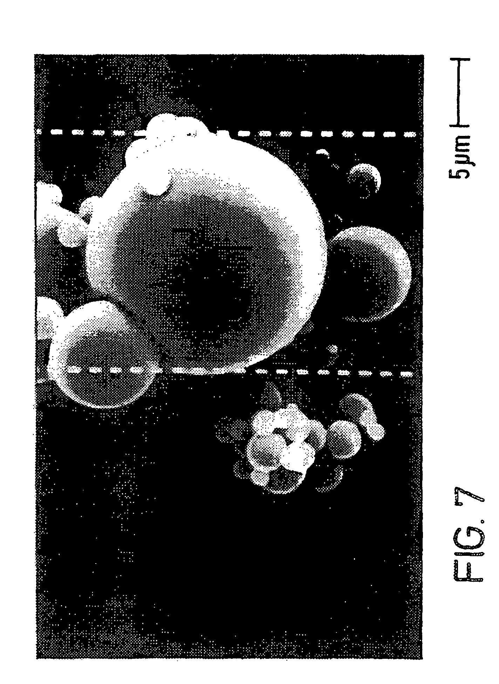

As noted in Table B and as can be seen in the photomicrographs in Figures 1 and 2, spray drying the Alb S produced dimpled solid structures and did not produce spherical hollow particulates. On the other hand, spray drying the Amil HCI produced spherical hollow particulates, as can be seen in the photomicrographs in Figures 3-5. Spray drying the lactose produced spherical hollow particulates, with the largest lactose particulate having an average particle diameter of about 17 μm, as can be seen in the photomicrographs in Figures 6 and 7.

While it is not intended to be bound to any theory, it is believed that the concentration of Alb S in water, namely a 5% solution of 15 g in 300 ml, was not low enough for the spray drying to result in spherical hollow particulates of Alb S, and thus, lowering the concentration of Alb S should result in spherical hollow particulates. Also, it is believed that admixing the Alb S with an excipient, such as lactose, during the spray drying should result in spherical hollow particulates.

Example II

Use of hollow particulates in dry powder inhalators.

The following is a discussion of how a DISKHALER™ (a medicament dispersing device, i.e., an inhalator, commercially available from GlaxoWellcome, Inc.) and an AEROBREATHER™ (available from API of Hadley, Massachusetts) may be employed with the spherical hollow medicament particulates of the present invention, such as the Amil HCI made in Example I, to determine how the powdered medicament is dispersed and thus illustrate that the spherical hollow medicament particulates are useful in a dry powder inhaler. More particularly, the extent to which a medicament is dispersed may be measured by its mass median aerodynamic diameter (MMAD) in micrometers, and the percentage that is less than 6 micrometers, particularly less than 5 micrometers, is indicative of desirable particle size for inhalation into the lungs.

Several DISKHALER™ devices should be employed. The DISKHALER™ has a screen which serves to direct an air jet, thus helping to entrain the particles in the air jet. The 4-blister compartment would be removed from the holder portion of each DISKHALER™.

A dosage of each of the spray dried spherical hollow particulates would be respectively loaded onto the bottom of the holder portion of a DISKHALER™, the bottom serving as a carrier surface. Next, each DISKHALER™ with its respective medicament would be attached to an AEROBREATHER™ for dispersion of the medicament from the carrier. The AEROBREATHER™ is a device that simulates inspiration by a human through the mouth at 60 liters/minute, with an acceleration of 19 liters/second$^2$ and a total volume of 1 liter.

The inspired powder (which would be approximately 1 milligram) then would be drawn into the AEROSIZER™ unit for aerodynamic particle size analysis. The photomultiplier tubes of the AEROSIZER™ would be operated at 1100 volts, and the data would be analyzed in an auto-combine mode with software version 5.02.37 available from API of Hadley, Massachusetts. As noted above, the extent to which the powder is dispersed is measured by the MMAD in micrometers, and the percentage that is less than 6 micrometers, particularly less than 5 micrometers, is indicative of desirable particle size for inhalation into the lungs.

The results for the dispersed spray dried spherical hollow medicament particulates should be a MMAD from about 0.5 to about 7 µm, particularly about 1 to about 4.5 µm, and a % mass < 6µm of about 30% or more, particularly about 50% or more, and most particularly about 70% or more. Also, the spherical hollow particulates of the present invention should be deposited deeper in the lungs than are conventional micronized solid particulates (with substantially the same geometric diameter) from a dry powder inhalator.

Example III

Use of hollow particulates in pressurized aerosol metered dose inhalators.

The following is a discussion of how an inhalator that is a pressurized aerosol container with a valve may be employed with the spherical hollow medicament particulates of the present invention, such as the Amil HCl made in Example I.

Example formulations suitable for a metered dose inhalator according to this invention include (I) a suspension consisting essentially of spherical hollow medicament particulates of respirable size and 1,1,1,2-tetrafluoroethane; and (ii) a suspension of spherical hollow medicament particulates of respirable size, 1,1,1,2-tetrafluoroethane, oleic acid and sufficient ethanol to solubulize the oleic acid.

The spherical hollow medicament particulates should be added to a high shear blender (i.e., mixer) which contains, for instance, 1,1,1,2-tetrafluoroethane propellant (colloquially known under the trade name, HFC-134a) and lecithin suspending agent.

However, the vapor pressure of 1,1,1,2-tetrafluoro-ethane propellant at 68°F is 68.4 psig, and hence, the vapor pressure is too great to meet the U.S. Government Department of Transportation requirements for use in aerosol containers when the containers are transported and temperatures can go up to 130°F. Thus, a vapor pressure depressant, such as a glycol ether (i.e., 2-butoxyethanol) or an alkyl acetate (i.e., butyl acetate) should be used together with 1,1,1,2-tetrafluoroethane propellant so that the resultant suspension in the aerosol container meets the Department of Transportation requirements and has a vapor pressure of less than 180 psig at 130°F.

Also, since 1,1,1,2-tetrafluoroethane propellant has a low boiling point of -15.5°F (-26.5°C) so that it would volatilize during procedures at or near room temperature, then the mixer should be maintained well below room temperature to prevent evaporation. Alternatively, a sealed mixer (one in a closed system with the container) may be employed.

Once a homogenous suspension is obtained, it is filled into aerosol containers. During the filling, the mixer can be used to maintain adequate suspension throughout the entire filling circuit by continuously circulating the suspension through the concentrated filling unit.

Because, as noted, 1,1,1,2-tetrafluoroethane propellant has a high vapor pressure, care must be taken during filling as filling through the valve of the container is difficult with such high pressure propellants. With one technique, the entire formulation is prepared in a low temperature pressure vessel and then filled through the valve into evacuated, previously crimped containers.

With an alternative technique, the propellant is not placed in suspension with the medicament and suspending agent prior to filling. Rather, filling of the suspension of medicament and suspending agent into each container is accomplished in a controlled environment, after which the valve is crimped in place onto the containers. Subsequently, the high pressure 1,1,1,2-tetrafluoroethane propellant is added through the valve.

As noted above, the extent to which a medicament is dispersed may be measured by its mass median aerodynamic diameter (MMAD) in micrometers, and the percentage that is less than 6 micrometers, particularly less than 5 micrometers, is indicative of desirable particle size for inhalation into the lungs.

Accordingly, like the results noted above in Example II for the spray dried spherical hollow medicament particulates dispersed from a dry powder inhalator, the results for the spray dried spherical hollow medicament particulates dispersed from pressurized aerosol containers should be a MMAD from about 0.5 to about 7 µm, particularly about 1 to about 4.5 µm, and a % mass < 6µm of about 30% or more, particularly about 50% or more, and most particularly about 70% or more. Also, the spherical hollow particulates of the present invention should be deposited deeper in the lungs than are conventional micronized solid particulates (with substantially the same geometric diameter) from an aerosol inhalator.

**Claims**

1. An inhalator device comprising an inhalator which contains at (east one dose of medicament particles comprising spherical hollow particulates that are of respirable particle size suitable for deposition in a human being's lungs.

2. The inhalator device of claim 1, wherein the spherical hollow particulates of respirable particle size have an average mass median aerodynamic diameter from about 0.5 μm to about 7.0 μm.

3. The inhalator device of claim 1 or 2, wherein the spherical hollow particulates of respirable particle size have a mass median aerodynamic diameter from about 1 μm to about 4.5 μm.

4. The inhalator device of any of claims 1 to 3, wherein the spherical hollow particulates of respirable particle size have more than about 50% thereof with a mass median aerodynamic diameter less than about 6 μm.

5. The inhalator device of any of claims 1 to 4, wherein the spherical hollow particulates of respirable particle size have more than about 70% thereof with a mass median aerodynamic diameter less than about 6 μm.

6. The inhalator device of any of claims 1 to 5, wherein the spherical hollow particulates of respirable particle size have an average geometric diameter of greater than 10 microns.

7. The inhalator device of any of claims 1 to 6, wherein the medicament is an analgesic, anginal preparation, antiallergic, anti-infective, anti-histamine, antiinflammatory agent, antitussive, bronchodilator, diuretic, anticholinergic, hormone, xanthine, or therapeutic protein or peptide.

8. The inhalator device of any of claims 1 to 7, wherein the medicament further includes therewith an excipient selected from the group consisting of polysaccharides, amino acids, lactose, glucose, mannitol, maltodextrins, calcium carbonate, sodium chloride and combinations thereof.

9. The inhalator device of any of claims 1 to 8, wherein the inhalator comprises a dry powder inhalator including a medicament carrier adapted for holding at least one dose of medicament and the spherical hollow medicament particulates are in a dry powder form and loaded in the carrier.

10. The inhalator device of any of claims 1 to 7, wherein the inhalator comprises a pressurized aerosol container having a dispensing valve and being adapted for containing at least one dose of medicament and the spherical hollow medicament particulates are in a suspension form with a propellant and the pressurised aerosol container contains therein the suspension.

11. The inhalator device of claim 10, wherein the propellant is selected from the group consisting of a chlorofluorocarbon, an environmentally safe propellant, and combinations thereof.

12. The inhalator device of claim 11, wherein the propellant is an environmentally safe propellant selected from the group consisting of perfluoroethane, 1,1-difluoroethane, monochlorodifluoromethane, 1,1,1,2-tetrafluoroethane, and combinations thereof.

13. The inhalator device of any of claims 10 to 12, wherein the pressurized aerosol container further contains therein a suspending agent and wherein the spherical hollow medicament particulates are in a suspension with the suspending agent.

14. The inhalator device of claim 13, wherein the suspending agent is selected from the group consisting of oleic acid, partial esters of common fatty acids and hexitol anhydrides, lecithin, and combinations thereof.

15. The inhalator device of any of claims 10 to 14, wherein the medicament particulates and the propellant have substantially the same density.

16. The inhalator device of any of claims 1 to 7, wherein the inhalator comprises a dry powder inhalator containing at least one dose of pure medicament formed of spherical hollow medicament particulates in dry powder form.

17. A medicament formulation in suspension form comprising spherical hollow medicament particulates of restorable size and a propellant selected from the group consisting of a chlorofluorocarbon, perfluoroethane, 1,1-difluoroethane, monochlorodifluoromethane, 1,1,2-tetrafluoroethane and any combinations thereof.

18. The medicament formulation of claim 17, wherein the medicament is selected from the group consisting of albuterol, amiloride, terbutaline, isoproterenol, metaprotaranol, pirbuterol, salmeterol, fluticasone propionate, budesonide, beclomethasone dipropionate, disodium cromoglycate, bambuterol, mometasone, ipratropium, insulin, triacetonide, and any pharmaceutically acceptable salts, solvates or esters thereof and any combinations thereof.

19. The medicament formulation of claim 17 or 18, additionally comprising a suspending agent is selected from the group consisting of oleic acid, partial esters of common fatty acids and hexitol anhydrides, lecithin, and combinations thereof.

20. The medicament formulation of any of claims 16 to 19, additionally comprising ethanol.

21. A medicament formulation in dry powder from comprising spherical hollow medicament particulates of respirable size, wherein the medicament is selected from the group consisting of amiloride, terbutaline, isoproterenol, metaprotaranol, pirbuterol, salmeterol, fluticasone propionate, budesonide, beclomethasone dipropionate, disodium cromoglycate, bambuterol, mometasone, ipratropium, triacetonide, and any pharmaceutically acceptable salts, solvates or esters thereof and any combinations thereof.

22. The medicament formulation of claim 21, wherein the medicament further includes therewith an excipient selected from the group consisting of polysaccharides, amino acids, lactose, glucose, mannitol, maltodextrins, calcium carbonate, sodium chloride and combinations thereof.

23. The medicament formulation of any of claims 17 to 22, wherein the spherical hollow particulates of respirable particle size have an average mass median aerodynamic diameter from about 0.5 µm to about 7.0 µm.

24. The medicament formulation of any of claims 17 to 23, wherein the spherical, hollow particulates of respirable particle size have an average mass median aerodynamic diameter from about 1 µm to about 4.5 µm.

25. The medicament formulation of any of claims 17 to 24, wherein the spherical hollow particulates of respirable particle size have more than about 50% thereof with a mass median aerodynamic diameter less than about 6 µm.

26. The medicament formulation of any of claims 17 to 25, wherein the spherical hollow particulates of respirable particle size have more than about 70% thereof with a mass median aerodynamic diameter less than about 6 µm.

27. The medicament formulation of any of claims 17 to 26, wherein the spherical hollow particulates of respirable particle size have an average geometric diameter of greater than 10 microns.

28. The medicament formulation of any of claims 17 - 27 wherein the medicament is salbutamol, salmeterol, fluticasone propionate, beclamethasone dipropionate, terbutaline, cromoglycate, budesonide and triamcinolone acetonide and/or salts thereof.

## Patentansprüche

1. Inhalatorvorrichtung, die einen Inhalator umfaßt, der wenigstens eine Dosis aus Medikamententeilchen enthält, die kugelförmige hohle Teilchen umfassen, die von atembarer Teilchengröße sind, die zur Abscheidung in den Lugen eines Menschen geeignet ist.

2. Inhalatorvorrichtung gemäß Anspruch 1, worin die kugelförmigen hohlen Teilchen von atembarer Teilchengröße einen durchschnittlichen aerodynamischen Massenmedian-Durchmesser von ca. 0,5 bis ca. 7,0 µm haben.

3. Inhalatorvorrichtung gemäß Anspruch 1 oder 2, worin die kugelförmigen hohlen Teilchen von atembarer Teilchengröße einen aerodynamischen Massenmedian-Durchmesser von ca. 1 bis ca. 4,5 µm haben.

4. Inhalatorvorrichtung gemäß einem der Ansprüche 1 bis 3, worin die kugelförmigen hohlen Teilchen von atembarer Teilchengröße mehr als ca. 50 % davon mit einem aerodynamischen Massenmedian-Durchmesser von weniger als ca. 6 µm aufweisen.

5. Inhalatorvorrichtung gemäß einem der Ansprüche 1 bis 4, worin die kugelförmigen hohlen Teilchen von atembarer Teilchengröße mehr als ca. 70 % davon mit einem aerodynamischen Massenmedian-Durchmesser von weniger als ca. 6 µm aufweisen.

**6.** Inhalatorvorrichtung gemäß einem der Ansprüche 1 bis 5, worin die kugelförmigen hohlen Teilchen von atembarer Teilchengröße einen durchschnittlichen geometrischen Durchmesser von mehr als 10 µm haben.

**7.** Inhalatorvorrichtung gemäß einem der Ansprüche 1 bis 6, worin das Medikament ein Analgetikum, eine Anginazubereitung, ein Antiallergikum, ein infektionsverhinderndes Mittel, ein Antihistaminikum, ein entzündungshemmendes Mittel, ein Antihustenmittel, ein Bronchodilatator, ein Diuretikum, ein Anticholinergikum, ein Hormon, ein Xanthin oder ein therapeutisches Protein oder Peptid ist.

**8.** Inhalatorvorrichtung gemäß einem der Ansprüche 1 bis 7, worin das Medikament ferner darin einen Hilfsstoff einschließt, der aus der Gruppe ausgewählt ist, die aus Polysacchariden, Aminosäuren, Lactose, Glucose, Mannit, Maltodextrinen, Calciumcarbonat, Natriumchlorid und Kombinationen daraus besteht.

**9.** Inhalatorvorrichtung gemäß einem der Ansprüche 1 bis 8, worin der Inhalator einen Trockenpulverinhalator umfaßt, der einen Medikamententräger einschließt, der zum Halten wenigstens einer Dosis von Medikament angepaßt ist, und die kugelförmigen hohlen Medikamententeilchen in einer Trockenpulverform sind und im Träger geladen sind.

**10.** Inhalatorvorrichtung gemäß einem der Ansprüche 1 bis 7, worin der Inhalator einen Druckaerosolbehälter umfaßt, der ein Spenderventil aufweist und dafür angepaßt ist, wenigstens eine Dosis von Medikament zu enthalten, und die kugelförmigen hohlen Medikamententeilchen in einer Suspensionsform mit einem Treibmittel sind und der Druckaerosolbehälter darin die Suspension enthält.

**11.** Inhalatorvorrichtung gemäß Anspruch 10, worin das Treibmittel aus der Gruppe ausgewählt ist, die aus Chlorfluorkohlenstoff, einem umweltverträglichen Treibmittel und Kombinationen daraus besteht.

**12.** Inhalatorvorrichtung gemäß Anspruch 11, worin das Treibmittel ein umweltverträgliches Treibmittel ist, das aus der Gruppe ausgewählt ist, die aus Perfluorethan, 1,1-Difluorethan, Monochlordifluormethan, 1,1,1,2-Tetrafluorethan und Kombinationen daraus besteht.

**13.** Inhalatorvorrichtung gemäß einem der Ansprüche 10 bis 12, worin der Druckaerosolbehälter ferner darin ein Suspendiermittel enthält, und worin die kugelförmigen hohlen Medikamententeilchen in einer Suspension mit dem Suspendiermittel sind.

**14.** Inhalatorvorrichtung gemäß Anspruch 13, worin das Suspendiermittel aus der Gruppe ausgewählt ist, die aus Oleinsäure, Partialestern von üblichen Fettsäuren und Hexitolanhydriden, Lecithin und Kombinationen daraus besteht.

**15.** Inhalatorvorrichtung gemäß einem der Ansprüche 10 bis 14, worin die Medikamententeilchen und das Treibmittel im wesentlichen die gleiche Dichte haben.

**16.** Inhalatorvorrichtung gemäß einem der Ansprüche 1 bis 7, worin der Inhalator einen Trockenpulverinhalator umfaßt, der wenigstens eine Dosis aus reinem Medikament enthält, gebildet aus kugelförmigen hohlen Medikamententeilchen in Trockenpulverform.

**17.** Medikamentenformulierung in Suspensionsform, die kugelförmige hohle Medikamententeilchen von atembarer Größe und ein Treibmittel umfaßt, das aus der Gruppe ausgewählt ist, die aus einem Chlorfluorkohlenstoff, Perfluorethan, 1,1-Difluorethan, Monochlordifluormethan, 1,1,1,2-Tetrafluorethan und beliebigen Kombinationen daraus besteht.

**18.** Medikamentenformulierung gemäß Anspruch 17, worin das Medikament aus der Gruppe ausgewählt ist, die aus Albuterol, Amilorid, Terbutalin, Isoproterenol, Metaprotaranol, Pirbuterol, Salmeterol, Fluticasonpropionat, Budesonid, Beclomethasondipropionat, Dinatriumcromoglycat, Bambuterol, Mometason, Ipratropium, Insulin, Triacetonid und beliebigen pharmazeutisch akzeptablen Salzen, Sovaten oder Estern daraus und beliebigen Kombinationen daraus besteht.

**19.** Medikamentenformulierung gemäß Anspruch 17 oder 18, die zusätzlich ein Suspendiermittel umfaßt, das aus der Gruppe ausgewählt ist, die aus Oleinsäure, Partialestern von üblichen Fettsäuren und Hexitolanhydriden, Lecithin und Kombinationen daraus besteht.

**20.** Medikamentenformulierung gemäß einem der Ansprüche 16 bis 19, die zusätzlich Ethanol umfaßt.

**21.** Medikamentenformulierung in Trockenpulverform, die kugelförmige hohle Medikamententeilchen von atembarer Größe umfaßt, worin das Medikament aus der Gruppe ausgewählt ist, die aus Amilorid, Terbutalin, Isoproterenol, Metaprotaranol, Pirbuterol, Salmeterol, Fluticasonpropionat, Budesonid, Beclomethasondipropionat, Dinatriumcromoglycat, Bambuterol, Mometason, Ipratropium, Triacetonid und beliebigen pharmazeutisch akzeptablen Salzen, Solvaten oder Estern daraus und beliebigen Kombinationen daraus besteht.

**22.** Medikamentenformulierung gemäß Anspruch 21, worin das Medikament ferner darin einen Hilfsstoff einschließt, ausgewählt aus der Gruppe, die aus Polysacchariden, Aminosäuren, Lactose, Glucose, Mannit, Maltodextrinen, Calciumcarbonat, Natriumchlorid und Kombinationen daraus besteht.

**23.** Medikamentenformulierung gemäß einem der Ansprüche 17 bis 22, worin die kugelförmigen hohlen Teilchen von atembarer Teilchengröße einen durchschnittlichen aerodynamischen Massenmedian-Durchmesser von ca. 0,5 bis ca. 7,0 µm haben.

**24.** Medikamentenformulierung gemäß einem der Ansprüche 17 bis 23, worin die kugelförmigen hohlen Teilchen von atembarer Teilchengröße einen durchschnittlichen aerodynamischen Massenmedian-Durchmesser von ca. 1 bis ca. 4,5 µm haben.

**25.** Medikamentenformulierung gemäß einem der Ansprüche 17 bis 24, worin die kugelförmigen hohlen Teilchen von atembarer Teilchengröße mehr als ca. 50 % davon mit einem aerodynamischen Massenmedian-Durchmesser von weniger als ca. 6 µm aufweisen.

**26.** Medikamentenformulierung gemäß einem der Ansprüche 17 bis 25, worin die kugelförmigen hohlen Teilchen von atembarer Teilchengröße mehr als ca. 70 % davon mit einem aerodynamischen Massenmedian-Durchmesser von weniger als ca. 6 µm aufweisen.

**27.** Medikamentenformulierung gemäß einem der Ansprüche 17 bis 26, worin die kugelförmigen hohlen Teilchen von atembarer Teilchengröße einen durchschnittlichen geometrischen Durchmesser von mehr als 10 µm haben.

**28.** Medikamentenformulierung gemäß einem der Ansprüche 17 bis 27, worin das Medikament Salbutamol, Salmeterol, Fluticasonpropionat, Beclamethasondipropionat, Terbutalin, Cromoglycat, Budesonid und Triamcinolonacetonid und/Salze davon ist.


**Revendications**

**1.** Dispositif d'inhalation comprenant un inhalateur contenant au moins une dose de particules de médicament comprenant des particules creuses sphériques de taille particulaire respirable appropriées pour être déposées dans les poumons d'un être humain.

**2.** Dispositif d'inhalation selon la revendication 1, dans lequel les particules creuses sphériques de taille particulaire respirable ont un diamètre massique médian aérodynamique moyen d'environ 0,5 µm à environ 7,0 µm.

**3.** Dispositif d'inhalation selon la revendication 1 ou 2, dans lequel les particules creuses sphériques de taille particulaire respirable ont un diamètre massique médian aérodynamique d'environ 1 µm à environ 4,5 µm.

**4.** Dispositif d'inhalation selon l'une quelconque des revendications 1 à 3, dans lequel les particules creuses sphériques de taille particulaire respirable ont pour plus d'environ 50 % d'entre elles un diamètre massique médian aérodynamique inférieur à environ 6 µm.

**5.** Dispositif d'inhalation selon l'une quelconque des revendications 1 à 4, dans lequel les particules creuses sphériques de taille particulaire respirable ont pour plus d'environ 70 % d'entre elles un diamètre massique médian aérodynamique inférieur à environ 6 µm.

**6.** Dispositif d'inhalation selon l'une quelconque des revendications 1 à 5, dans lequel les particules creuses sphériques de taille particulaire respirable ont un diamètre géométrique moyen supérieur à 10 microns.

**7.** Dispositif d'inhalation selon l'une quelconque des revendications 1 à 6, dans lequel le médicament est un analgésique, une préparation angineuse, un antiallergique, un anti-infectieux, un antihistaminique, un agent anti-inflammatoire, un antitussif, un bronchodilatateur, un diurétique, un anticholinergique, une hormone, une xanthine ou une protéine ou un peptide thérapeutique.

**8.** Dispositif d'inhalation selon l'une quelconque des revendications 1 à 7, dans lequel le médicament inclut en outre un excipient choisi dans le groupe constitué par les polysaccharides, des acides aminés, du lactose, du glucose, du mannitol, des maltodextrines, du carbonate de calcium, du chlorure de sodium et des combinaisons de ceux-ci.

**9.** Dispositif d'inhalation selon l'une quelconque des revendications 1 à 8, dans lequel l'inhalateur comprend un inhalateur de poudre sèche comprenant un véhicule de médicament adapté à contenir au moins une dose de médicament et les particules creuses sphériques de médicament sont sous la forme d'une poudre sèche et sont chargées dans le véhicule.

**10.** Dispositif selon l'une quelconque des revendications 1 à 7, dans lequel l'inhalateur comprend un récipient d'aérosol pressurisé ayant une valve de distribution et étant adapté à contenir au moins une dose de médicament et les particules creuses sphériques de médicament sont sous la forme d'une suspension avec un propulseur et le récipient d'aérosol pressurisé contient la suspension.

**11.** Dispositif d'inhalation selon la revendication 10, dans lequel le propulseur est choisi dans le groupe constitué d'un chlorofluorocarbone, d'un propulseur écologique, et les combinaisons de ceux-ci.

**12.** Dispositif d'inhalation selon la revendication 11, dans lequel le propulseur est un propulseur écologique choisi dans le groupe constitué du perfluoroéthane, du 1,1-difluoroéthane, du monochlorodifluorométhane, du 1,1,1,2-tétrafluoroéthane, et les combinaisons de ceux-ci.

**13.** Dispositif d'inhalation selon l'une quelconque des revendications 10 à 12, dans lequel le récipient d'aérosol pressurisé contient en outre un agent de suspension et dans lequel les particules creuses sphériques de médicament sont en suspension avec l'agent de suspension.

**14.** Dispositif d'inhalation selon la revendication 13, dans lequel l'agent de suspension est choisi dans le groupe constitué de l'acide oléique, des esters partiels des acides gras communs et des anhydrides d'hexitol, de la lécithine, et des combinaisons de ceux-ci.

**15.** Dispositif d'inhalation selon l'une quelconque des revendications 10 à 14, dans lequel les particules de médicament et le propulseur ont sensiblement la même densité.

**16.** Dispositif d'inhalation selon l'une quelconque des revendications 1 à 7, dans lequel l'inhalateur comprend un inhalateur de poudre sèche contenant au moins une dose de médicament pur formé de particules creuses sphériques de médicament sous la forme de poudre sèche.

**17.** Formulation médicamenteuse sous la forme d'une suspension comprenant des particules creuses sphériques de médicament de taille reconstituable et un propulseur choisi dans le groupe constitué du chlorofluorocarbone, du perfluoroéthane, du 1,1-difluoroéthane, du monochlorodifluorométhane, du 1,1,2-tétrafluoroéthane et de toute combinaison de ceux-ci.

**18.** Formulation médicamenteuse selon la revendication 17, dans laquelle le médicament est choisi dans le groupe constitué de l'albutérol, de l'amiloride, de la terbutaline, de l'isoprotérénol, du métaprotaranol, du pirbutérol, du salmétérol, du propionate de fluticasone, du budésonide, du dipropionate de béclométhasone, du cromoglycate disodique, du bambutérol, du mométasone, de l'ipratropium, de l'insuline, du triacétonide, et de tout sel, solvant ou ester pharmaceutiquement acceptable de ceux-ci ainsi que de toute combinaison de ceux-ci.

**19.** Formulation médicamenteuse selon la revendication 17 ou 18, comprenant en outre un agent de suspension choisi dans le groupe constitué de l'acide oléique, des esters partiels des acides gras communs et des anhydrides d'hexitol, de la lécithine et des combinaisons de ceux-ci.

**20.** Formulation médicamenteuse selon l'une quelconque des revendications 16 à 19, comprenant en outre de l'éthanol.

**21.** Formulation médicamenteuse sous la forme de poudre sèche comprenant des particules creuses sphériques de médicament de taille respirable, dans laquelle le médicament est choisi dans le groupe constitué d'amiloride, de terbutaline, d'isoprotérénol, de métaprotaranol, de pirbutérol, de salmétérol, de propionate de fluticasone, de budésonide, de dipropionate de béclométhasone, de cromoglycate disodique, de bambutérol, de mométasone, d'ipratropium, de triacétonide, et de tout sel, solvant ou ester pharmaceutiquement acceptable de ceux-ci et de toute combinaison de ceux-ci.

**22.** Formulation médicamenteuse selon la revendication 21, dans laquelle le médicament comprend en outre un excipient choisi dans le groupe constitué par les polysaccharides, les acides aminés, le lactose, le glucose, le mannitol, les maltodextrines, le carbonate de calcium, le chlorure de sodium et les combinaisons de ceux-ci.

**23.** Formulation médicamenteuse selon l'une quelconque des revendications 17 à 22, dans laquelle les particules creuses sphériques de taille particulaire respirable ont un diamètre massique médian aérodynamique moyen d'environ 0,5 μm à environ 7,0 μm.

**24.** Formulation médicamenteuse selon l'une quelconque des revendications 17 à 23, dans laquelle les particules creuses sphériques de taille particulaire respirable ont un diamètre massique médian aérodynamique moyen d'environ 1 μm à environ 4,5 μm.

**25.** Formulation médicamenteuse selon l'une quelconque des revendications 17 à 24, dans laquelle les particules creuses sphériques de taille particulaire respirable ont pour plus d'environ 50 % d'entre elles un diamètre massique médian aérodynamique inférieur à environ 6 μm.

**26.** Formulation médicamenteuse selon l'une quelconque des revendications 17 à 25, dans laquelle les particules creuses sphériques de taille particulaire respirable ont pour plus d'environ 70 % d'entre elles un diamètre massique médian aérodynamique inférieur à environ 6 μm.

**27.** Formulation médicamenteuse selon l'une quelconque des revendications 17 à 26, dans laquelle les particules creuses sphériques de taille particulaire respirable ont un diamètre géométrique moyen supérieur à 10 microns.

**28.** Formulation médicamenteuse selon l'une quelconque des revendications 17 à 27 dans laquelle le médicament est du salbutamol, du salmétérol, du propionate de fluticasone, du dipropionate de béclaméthasone, de la terbutaline, du cromoglycate, du budésonide ou de l'acétonide de triamacinolone et/ou les sels de ceux-ci.

FIG. 1

5 μm

500nm

FIG. 2

FIG. 3

5 µm

2 μm

FIG. 4

500 nm

FIG. 5

FIG. 6

FIG. 7